# EUROPEAN PATENT SPECIFICATION

(11) **EP 4 037 756 B1**
(45) Date of publication and mention of the grant of the patent: **30.07.2025**
(21) Application number: 20792305.3
(22) Date of filing: 02.10.2020
(51) Int. Cl.: A61N 1/05

(54) **MEDICAL ELECTRODE DEVICE FOR IMPLANTATION INTO A PATIENT**
MEDIZINISCHE ELEKTRODENVORRICHTUNG ZUR IMPLANTATION IN EINEN PATIENTEN
DISPOSITIF D'ÉLECTRODE MÉDICALE À IMPLANTER DANS UN PATIENT

(30) Priority: 02.10.2019 US 201962909251 P
(43) Date of publication of application: 10.08.2022
(60) Divisional application: 25184373.6
(73) Proprietor: BIOTRONIK SE & Co. KG, 12359 Berlin (DE)
(72) Inventor: RIAHI, Pamela Shamsie Victoria, Portland, OR 97210 (US); KIBLER, Andrew B., Lake Oswego, OR 97035 (US); KAISER, Dajana, 12247 Berlin (DE); WILLENBERG, Patrick, 12459 Berlin (DE)
(74) Representative: Biotronik Corporate Services SE
(86) International application number: PCT/EP2020/077650
(87) International publication number: WO 2021/064168

(56) References cited:
- US-A1- 2002 128 700
- US-A1- 2005 154 435
- US-A1- 2007 150 036
- US-A1- 2017 246 452
- US-A1- 2018 296 823

## Description

The present invention relates to a medical electrode device for implantation into a patient according to claim 1 and to a method for fabricating a medical electrode device according to claim 14.

A medical electrode device of this kind may for example serve for neuro-stimulation and for this may be implanted into a patient for example in the region of the spinal cord, for example into the epidural space near the spinal cord of the spinal column of a patient. In this way nerve stimulation at the spinal cord may be achieved by injecting electrical current.

An electrode device of the kind concerned herein however may also be used for emitting stimulation signals or receiving sense signals at other locations within a patient, for example within the brain or for cardiac applications.

An electrode device of the kind concerned herein comprises a carrier element being formed from an electrically insulating material and defining a surface generally extending along a plane of extension, and at least one electrode element arranged on the carrier element for at least one of emitting an electrical signal and receiving an electrical signal. One or multiple electrode elements herein may be placed on the carrier element at the surface formed by the carrier element such that the electrode elements may be brought into electrical contact with surrounding tissue, such that electrical signals may be injected to or received from the tissue for achieving stimulation or sensing of signals.

The medical electrode device may have the shape of a so-called paddle electrode, the carrier element in this case having a planar paddle-like shape carrying e.g. an arrangement of multiple evenly or unevenly distributed electrode elements on its surface for emission of electrical signals into and/or reception of electrical signals from surrounding tissue.

There is a general desire to enable fabrication of a medical electrode device in an easy and cost-efficient manner.

In addition, there is a desire to optimize a current density profile on an electrode element of the electrode device to be able to emit and/or receive electrical signals in an efficient, sensitive manner. Generally, a current density on an electrode element may be substantially uneven, a current density profile potentially exhibiting pronounced peaks at edges of the electrode element, due to a so-called fringe or perimeter effect causing a substantial portion of the current flowing along the perimeter of the electrode element, hence giving rise to an increased current density at an edge region of the electrode element. As excessively high current densities may cause local tissue damage and may further promote electrode corrosion, there is a desire to design an electrode element of a medical electrode device such that an improved current density profile in particular for electrical stimulation of surrounding tissue is achieved.

US 6,052,608 discloses an electrode device used in particular for sensing cortical electrical activity. The electrode device herein comprises an arrangement of electrode elements having a semi-spherical shape, the electrode elements being surrounded by an electrically insulating material of a carrier element.

Different designs of paddle electrodes are known, for example, from US 6,895,283 and US 9,561,363.

US 2017/246452 A1 represents further background art.

It is an object of the instant invention to provide a medical electrode device and a method for fabricating a medical electrode device allowing for an easy and cost-efficient fabrication as well as an improved current density for electrical stimulation and/or sensing.

This object is achieved by means of a medical electrode device comprising the features of claim 1 and a method for fabricating a medical electrode device comprising the features of claim 14.

Herein, the at least one electrode element comprises a first wall section and a second wall section. The first wall section, in a cross-section along a cross-sectional plane perpendicular to said plane of extension, extends straight along a perpendicular direction with respect to said plane of extension or at an oblique angle with respect to said perpendicular direction, the first wall section being in contact with the electrically insulating material of the carrier element. The second wall section, in said cross-section along said cross-sectional plane perpendicular to said plane of extension, adjoins the first wall section and is curved, the second wall section not being in contact with the electrically insulating material of the carrier element.

For fabricating the medical electrode device, one or multiple electrode elements are arranged on the carrier element, the one or the multiple electrode elements being received on the carrier element and being at least partially embedded into the electrically insulating material of the carrier element. Herein, the one or the multiple electrode elements shall be held securely on the carrier element, and shell exhibit a beneficial electrical behavior in use of the medical electrode device, for example in combination with a medical stimulation device, such as a neuro-stimulation device.

For this, the electrode element in a cross-section along a cross-sectional plane perpendicular to the plane of extension of the surface of the carrier element, comprises a first wall section which faces towards the outside of the electrode element and is formed to extend straight along a perpendicular direction with respect to the plane of extension of the carrier element or at an oblique angle with respect to the perpendicular direction. E.g., the first wall section maybe extend from the second wall section in an oblique, outwardly flared direction. The first wall section is in contact with the material of the carrier element. By means of the first wall section the electrode element hence is embedded into the carrier element and in this way fastened to the carrier element.

It shall be noted in this respect that the carrier element generally extends along a plane of extension and hence may have a generally planar shape. The carrier element may be flat or may have a curved shape, in particular when the carrier element is formed from a material which at least to some extend is flexible, such as a silicone material. When it is referred to the perpendicular direction it hence is meant that the perpendicular direction is perpendicular to the generally planar extension of the carrier element in the vicinity of the respective electrode element.

The first wall section, in said cross-section, is adjoined by a second wall section, which faces towards the outside of the electrode element and extends from the first wall section towards and potentially beyond the surface of the carrier element. The second wall section is curved and (at its outside) is not in contact with the material of the carrier element, such that the second wall section at its outside is free and may provide for a beneficial emission of electrical stimulation signals into or a reception of electrical sense signals from surrounding tissue.

Due to the curved shape of the electrode element in the region of the second wall section a current density at edges of the electrode element may be reduced, such that the current density across the electrode element may be flattened by reducing current density peaks at the edges of the electrode element. Each edge of the electrode element herein may be formed by a corresponding second wall section, such that the electrode element comprises a generally rounded shape at a side facing towards the outside.

In the cross-section along the cross-sectional plane perpendicular to the plane of extension of the carrier element, herein, the electrode element may be bound, at opposite sides, by opposite first wall sections. Each first wall section at the opposite sides herein may be adjoined by an associated second wall section, the second wall section being curved such that the electrode element at its edges is curved, the curved edges herein not being in contact with the material of the carrier element, but being free from the material of the carrier element towards the outside.

By forming the electrode element, in said cross-section perpendicular to the plane of extension of the carrier element, to have a shape defined by perpendicular first wall sections and curved second wall sections, the electrode element may exhibit a simple shape and hence may be easy to fabricate, for example using a forming process, in particular a deep-drawing process to manufacture the electrode element from a metal sheet material.

The at least one electrode element comprises a third wall section adjoining the second wall section. The second wall section, in the cross-section along the cross-sectional plane perpendicular to the plane of extension of the carrier element, extends straight in a direction parallel to the plane of extension. The electrode element hence forms a straight face facing outwards which has a generally flat shape and extends in parallel to the generally surface of the carrier element.

By means of such a straight face formed by the third wall section an even contact of the electrode element with surrounding tissue may be achieved, the electrode element being enabled to come into contact with tissue across the entire electrode area that is not covered by material of the carrier element, but is exposed towards the outside. By the generally flat shape of the electrode element a contact pressure in between the electrode element and surrounding tissue may be evenly distributed, hence reducing the overall contact pressure in between the electrode element and surrounding tissue.

In one embodiment, the at least one electrode element protrudes, along the perpendicular direction, from the carrier element such that the at least one electrode element reaches beyond the surface of the carrier element. The electrode element hence, with a portion, is exposed towards the outside, edges of the electrode element being formed by curved second wall sections herein being free from the material of the carrier element, such that the edges may come into contact with the surrounding tissue. By having the electrode element protrude beyond the surface of the carrier element, a beneficial contact of the electrode element in particular also in edge regions of the electrode element (i.e. in the regions of the second wall sections forming the edges of the electrode element) may be achieved.

In one embodiment, the second wall section, in the cross-section along said cross-sectional plane perpendicular to the plane of extension of the carrier element, comprises a curved portion having an angular width and a (constant) radius of curvature across said angular width. The curved section hence may evenly be curved, the curvature being defined by the radius of curvature as measured in the cross-section along said cross-sectional plane. The second wall section may link the first, perpendicular wall section to the third, horizontal wall section, the angular width of the second wall section hence being for example 90°.

The radius of curvature herein may for example be equal to or larger than 0.5 mm, in particular equal to or larger than 0.8 mm, for example 1 mm. By forming the second wall section with a fairly large radius of curvature the electrode element assumes an approximate dome shape having a rounded dome facing towards the outside.

In one embodiment, the second wall section, in the cross-section along the cross-sectional plane perpendicular to the plane of extension of the carrier element, comprises a first curved portion and a second curved portion having a different curvature than the first curved portion. The second curved portion herein adjoins the first wall section. In particular, the first curved portion may comprise a first angular width and a first radius of curvature across the first angular width, and the second curved portion may comprise a second angular width and a second radius of curvature across the second angular width. In one embodiment, the first radius of curvature is larger than the second radius of curvature.

Hence, the second wall section comprises different portions having a different curvature. One portion herein may for example be curved according to a smaller radius of curvature than the other portion. The portion having the smaller radius of curvature herein may adjoin the first wall section and hence may point into the carrier element, whereas the other portion having the larger radius of curvature may adjoin the third wall section and hence may form a rounded transition towards the flat outer face of the electrode element.

In one embodiment, the first radius of curvature may be equal to or larger than 0.5 mm, in particular equal to or larger than 0.8 mm, for example 1 mm. The second radius of curvature may for example be equal to or larger than 0.1 mm, for example equal to or larger than 0.2 mm, for example 0.3 mm.

By forming the second wall section to have different portions exhibiting different curvatures, fabrication of the electrode element may be eased. Herein, due to the larger radius of curvature at the outer portion a beneficial, flattened current density may be obtained, due to an approximate rounded dome shape of the electrode element at a side facing towards the outside.

In one embodiment, the carrier element comprises a body portion and an overlapping portion.

The carrier element generally may form a recess in which the electrode element is received. The recess may be bound circumferentially by the body portion, the body portion contacting the first wall section of the electrode element such that the electrode element is securely fastened to the carrier element by connection of the first wall section to the body portion.

The overlapping portion, in turn, adjoins the body portion in the direction of the surface of the carrier element, the overlapping portion overlapping with the second wall section of the electrode element along the perpendicular direction. The overlapping portion herein does not contact the second wall section, such that the second wall section of the electrode element is free from the material of the carrier element. Hence, in this way, rounded edges of the electrode element are not covered by material of the carrier element, but are free to contact with surrounding tissue.

In one embodiment, the overlapping portion, in the cross-section along the cross-sectional plane perpendicular to the plane of extension, extends at an angle with respect to the perpendicular direction. The overlapping portion in particular may be angled away from the second wall section of the electrode element, such that a space around the second wall section and hence around the electrode element widens along the perpendicular direction towards the outside of the electrode element to allow for an easy contact of the electrode element in the region of the second wall section with surrounding tissue.

The angle formed by the overlapping portion may for example lie in a range between 30° and 60°, for example at 45°, with respect to the perpendicular direction.

In one embodiment, the at least one electrode element, in a cross-section parallel to the plane of extension of the carrier element, comprises a generally rectangular shape. The electrode element herein may for example have a length in a range between 1 mm and 10 mm, for example in between 2 mm and 5 mm, for example 3.5 mm, and a width (measured transverse to the length of the generally rectangular electrode element) in a range between 0.5 mm and 5 mm, for example in between 1 mm and 3 mm, for example 2 mm. Edges of the generally rectangular electrode element herein may be formed by second wall sections extending along the outer walls of the electrode element, the edges of the electrode element hence being rounded to obtain an improved, beneficially substantially even current distribution across the electrode element.

The at least one electrode element, in one embodiment, is formed from a metal material, in particular a platinum-iridium alloy, for example a 90:10, 80:20 or 70:30 platinum-iridium alloy. The electrode element may be formed in an easy and cost-efficient process using a deep-drawing technique. The electrode element including its cross-sectional wall sections hence may be formed in a forming process, wherein the electrode element may be cut from a metal sheet, in particular a platinum-iridium alloy sheet, and subsequently may be formed by deep-drawing in a forming tool. The electrode element herein may for example comprise a contact for contacting the electrode element with a supply line such that the electrode element may be electrically contacted and supplied within the electrode device.

The carrier element is formed from an electrically insulating material. The carrier element in particular may be formed from a silicone material, the material partially embedding the electrode element such that the material of the carrier element contacts with the perpendicular or oblique first wall section formed on the electrode element.

In another aspect a method for fabricating a medical electrode device for implantation into a patient is provided, the method comprising: forming a carrier element from an electrically insulating material such that the carrier element defines a surface generally extending along a plane of extension; forming, using a deep-drawing process, at least one electrode element for at least one of emitting an electrical signal and receiving an electrical signal; and arranging said at least one electrode element on said carrier element. Herein, said forming the at least one electrode element includes: forming the at least one electrode element to comprise a first wall section and a second wall section adjoining the first wall section. Further, the arranging step includes: arranging said at least one electrode element on said carrier element such that the first wall section is in contact with the electrically insulating material of the carrier element and the second wall section is not in contact with the electrically insulating material of the carrier element, wherein said first wall section, in a cross-section along a cross-sectional plane perpendicular to said plane of extension, extends straight along a perpendicular direction with respect to said plane of extension or at an oblique angle with respect to said perpendicular direction, and wherein said second wall section in said cross-section is curved.

The advantages and advantageous embodiments described above for the medical electrode device equally apply also to the method, such that it shall be referred to the above in this respect.

The various features and advantages of the present invention may be more readily understood with reference to the following detailed description and the embodiments shown in the drawings. Herein,
- Fig. 1: shows a view of an electrode device connected to a stimulation device in an implanted state in the area of the spine of a patient;
- Fig. 2: shows a view of the electrode device in the epidural space in the region of the spinal column;
- Fig. 3: shows a view of a flattened end of an embodiment of an electrode device;
- Fig. 4: shows a view of another embodiment of an electrode device;
- Fig. 5: shows a cross-sectional view of an electrode element of the electrode device embedded in a carrier element of the electrode device;
- Fig. 6: shows a view of an embodiment of an electrode element;
- Fig. 7A: shows a top view of the electrode element;
- Fig. 7B: shows a cross-sectional view along line A-A according to Fig. 7A;
- Fig. 7C: shows a cross-sectional view along line B-B according to Fig. 7A;
- Fig. 8A: shows a cross-sectional view of an embodiment of an electrode element;
- Fig. 8B: shows a cross-sectional view of another embodiment of an electrode element;
- Fig. 9: shows a current density profile obtained for the different embodiments of the electrode elements according to Figs. 8A and 8B;
- Fig. 10A-10F: show different embodiments of electrode elements embedded in material of a carrier element of the electrode device;
- Fig. 11: shows current densities as obtained for the different electrode elements according to Figs. 10A to 10F; and
- Fig. 12: shows a view of an electrode element according to another embodiment.

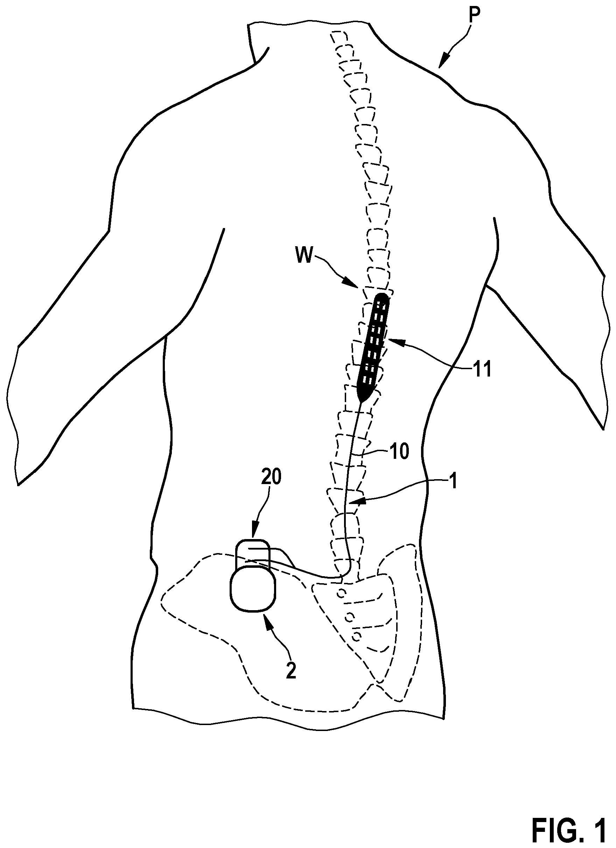

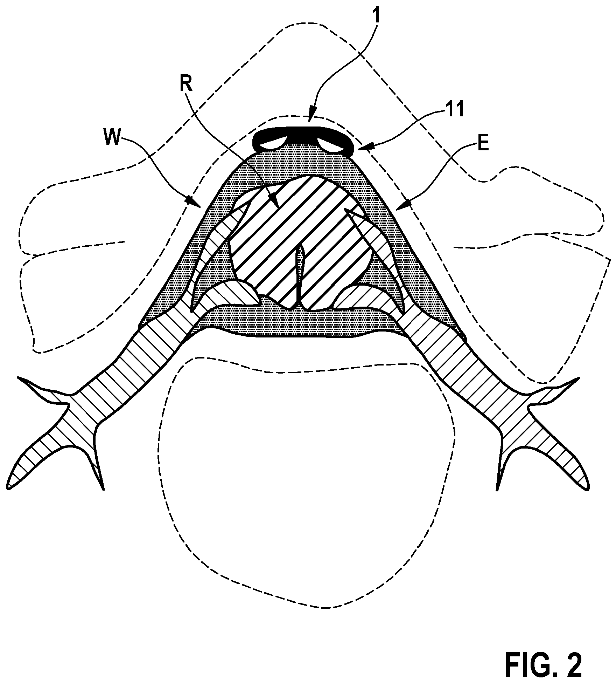

An electrode device 1, as shown in an embodiment in Figs. 1 and 2, is formed as a so-called paddle electrode and comprises an electrode body 10 and an electrode end 11 connected to the electrode body 10, a plurality of electrode elements being attached to the electrode end 11 for injecting electrical current e.g. in the region of the spinal column W of a patient P.

The electrode device 1 at a proximal end of the electrode body 10 is connected to a connector block 20 of a stimulation device 2, via which stimulation energy can be delivered to the electrode device 1 and radiated via the electrode arrangement arranged on the electrode end 11 to stimulate the spinal cord R in the region of the spinal column W.

As can be seen from the sectional view of Fig. 2, in the embodiment shown the electrode device 1 is implanted with the electrode end 11 in the epidural space E in the region of the spinal column W of the patient P in such a way that the electrode end 11 is located in the region of the spinal cord R and can thus introduce stimulation energy in a directed manner into the spinal cord R in order to effect nerve stimulation in the region of the spinal cord R.

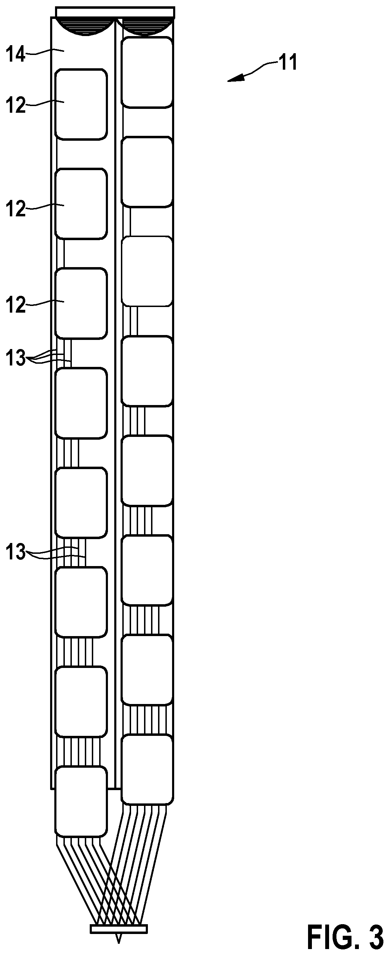

While the electrode body 10 for example comprises a circular (isodiametric) cross-section, the electrode device 1 is flattened in the area of the electrode end 11 which, as can be seen in Fig. 3, carries a plurality of electrode elements 12, which may be evenly or unevenly spaced on the electrode end 11 in such a way that stimulation energy can be fed in a directed manner for example into the spinal cord R of a patient P.

As further illustrated in Fig. 3, each electrode element 12 is connected to a supply line 13, wherein each electrode element 12 can be connected to the stimulation device 2 via an associated, individual supply line 13 and thus may be supplied with stimulation energy via the stimulation device 2 to emit an electrical signal. The supply lines 13 are jointly routed as a cable strand in the electrode body 10 in an encapsulated manner to the stimulation device 2.

The electrode elements 12 are arranged on a carrier 14, but are exposed with a surface facing outwards and can therefore come into contact with surrounding tissue when the electrode device 1 is implanted in a patient.

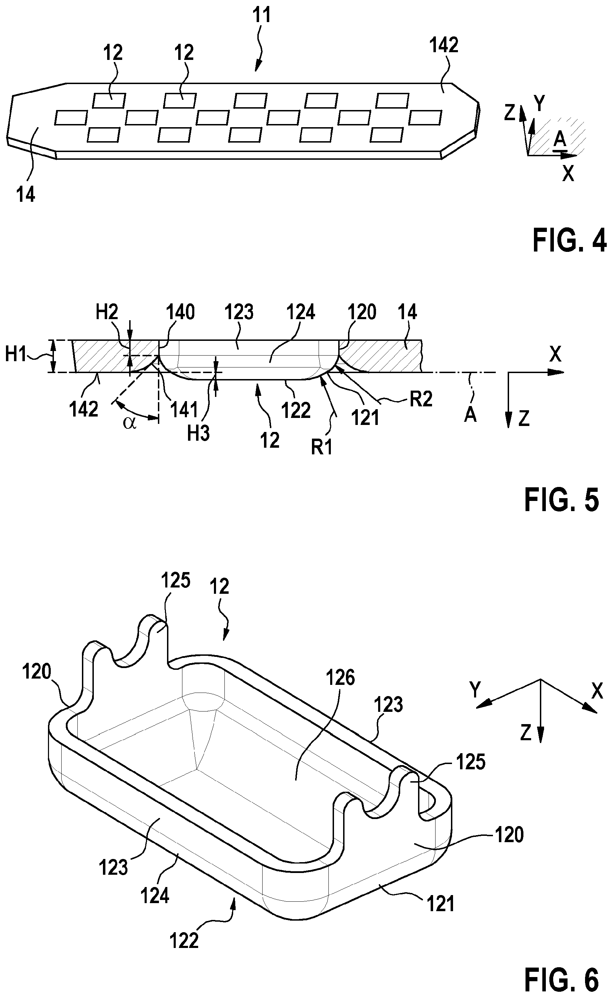

Fig. 4 shows another embodiment of an electrode device 1 having an electrode end 11 comprising a flattened, paddle-like shape, the electrode end 11 being formed by a carrier element 14 across which an arrangement of electrode elements 12 is evenly distributed to effect an emission of electrical stimulation signals into or allow for a sensing of electrical sense signals from surrounding tissue. As visible from Fig. 4, the electrode elements 12 are exposed towards the outside, such that faces of the electrode elements 12 are not covered with material of the carrier element 14, the carrier element 14 forming a surface 142 from which the electrode elements 12 are exposed.

The surface 142 extends in a planar fashion along a plane of extension A spanned by horizontal directions X, Y, as indicated in Fig. 4. The carrier element 14 herein is formed from an electrically insulating material, in particular a silicone material.

Referring now to Figs. 5 to 7A to 7C, in one embodiment one or multiple of the electrode elements 12, beneficially all of the electrode elements 12 of the electrode device 1 have a rounded, dome-like shape, as this is visible for example from the cross-sectional view of Fig. 5. The electrode element 12 is partially embedded within the material of the carrier element 14, such that the carrier element 12 is connected to the carrier element 14 by means of a material-fit connection.

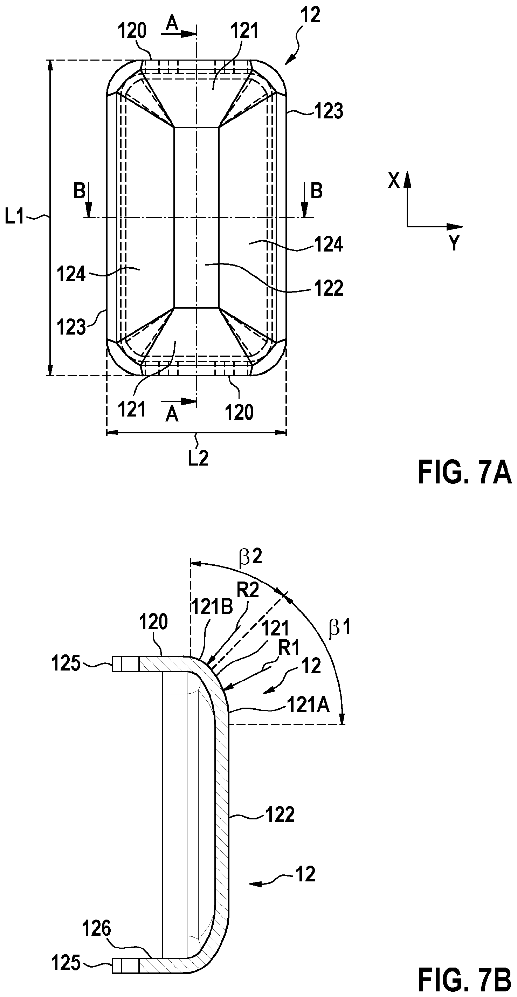

As visible from Figs. 6 and 7A to 7C, the electrode element 12 comprises a generally rectangular shape, forming opposite, short wall sections 120 and opposite, long wall sections 123. The wall sections 120 and the wall sections 123 are arranged at right angles with respect to each other to form the generally rectangular shape of the electrode element 12. Transitions between the wall sections 120, 123 herein are rounded, as visible from the top view of Fig. 7A.

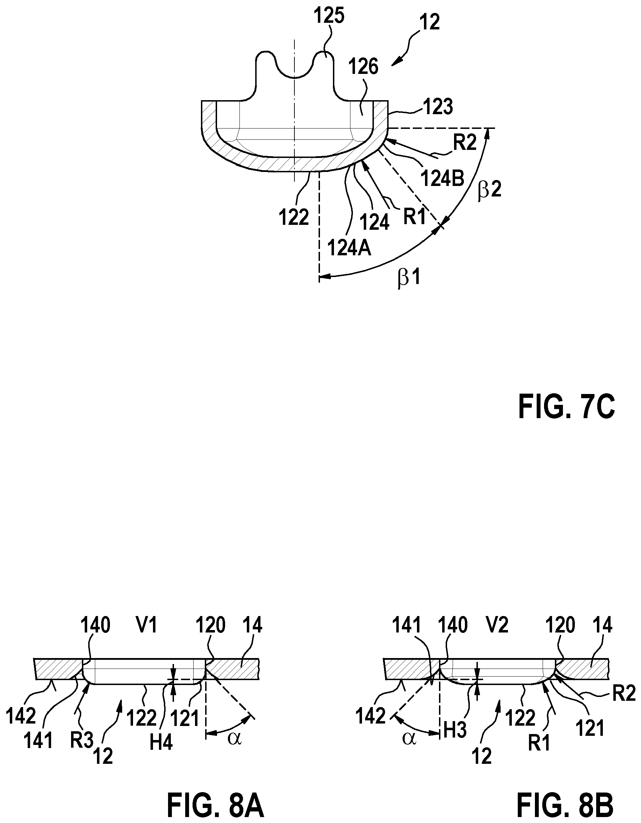

As visible from the cross-sectional views of Figs. 5 and 7B and 7C, the wall sections 120, 123 - in the shown embodiment - extend along a perpendicular direction Z with respect to the surface 142 of the carrier element 14 and hence perpendicularly to the plane of extension A of the carrier element 14. The wall sections 120, 123, as visible from Fig. 5, are in contact with surrounding body portions 140 of the carrier element 14 and hence form a material-fit connection with the carrier element 14.

The wall sections 120, 123 - in the cross-sectional planes of Fig. 5 and Figs. 7B and 7C - each are adjoined by a curved wall section 121, 124, the curved wall sections 121, 124 forming rounded edges of the electrode element 12 converging into a flat, straight wall section 122 of the electrode element 12 facing towards the outside, as it is visible for example from Fig. 5. The curved wall sections 121, 124 hence form a rounded transition in between the perpendicular, straight wall sections 120, 123 and the flat, horizontal wall section 122 extending in parallel to the plane of extension A and facing towards the outside.

The curved wall sections 121, 124 in the shown embodiment are made up of different portions 121A, 121B, 124A, 124B when viewed along the cross-sectional planes as indicated by lines A-A and B-B in Fig. 7A, the different portions 121A, 121B, 124A, 124B being defined by different radii of curvature R1, R2 and extending across different angular widths β1, β2, as illustrated in Fig. 7B and 7C.

In particular, a portion 121B, 124B having a radius of curvature R2, in the cross-sectional views of Fig. 7B and 7C, adjoins the associated straight wall section 120, 123 and spans across an angular width β2. Another portion 121A, 124A adjoins the portion 121B, 124B and comprises a radius of curvature R1 being substantially larger than the radius of curvature R2, the portion 121A, 124A spanning across an angular width β1 and transitioning into the flat, horizontal wall section 122.

Due to the different radii of curvature R1, R2 each rounded wall section 121, 124 comprises a tighter curvature at the transition to the associated, perpendicular wall section 120, 123, and a substantially lesser curvature at the transition into the third, outer face 122.

The radius of curvature R2 may be equal to or larger than 0.1 mm, for example equal to or larger than 0.2 mm, for example 0.3 mm.

The radius of curvature R1 may be equal to or larger than 0.5 mm, in particular equal to or larger than 0.8 mm, for example 1 mm.

The electrode element 12 may for example have a length L1, as illustrated in Fig. 7A, in between 2 mm and 5 mm, for example 3.5 mm, and a width L2 for example in between 1 mm and 3 mm, for example 2 mm.

As visible from Fig. 5, the electrode element 12 protrudes from the carrier element 14 in that the flat, horizontal wall section 122 is placed at a height H3 beyond the surface 142 of the carrier element 14. The electrode element 12 may comprise an overall height H1 (measured along the vertical direction Z, without the contact elements 125). The straight, perpendicular wall sections may comprise a height H2, as illustrated in Fig. 5, approximately half the overall height H1.

As visible from Fig. 5, the body portion 140 of the carrier element 14 being in contact with the straight, perpendicular wall sections 120, 123 of the electrode element 12 is adjoined by an associated overlapping portion 141, the overlapping portion 141 overlapping with the curved wall sections 121, 124 in the perpendicular direction Z, as visible from Fig. 5. Herein, in the shown embodiment, the overlapping portion 141 is arranged at an angle α with respect to the perpendicular direction Z, the angle α lying for example in a range between 30° and 60°, for example at 45°.

By means of the angled overlapping portion 141, which does not contact the associated curved wall sections 121, 124, the electrode element 12 with its curved edges formed by the curved wall sections 121, 124 and the flat, outer wall section 122 is exposed towards the outside and may come into contact with surrounding tissue.

In addition, due to the shaping of the electrode element 12, in particular with the differently curved portions 121A, 121B, 124A, 124B of the curved wall sections 121, 124, an easy manufacturing process may be used, allowing for example a fabrication of the electrode element 12 using a deep-drawing process.

In a deep-drawing process the electrode element 12 is formed from a sheet material using a stamping tool, the electrode element 12, as visible from Fig. 6, forming a recess 126 on its inside. Prior to forming the electrode element 12 by means of a deep-drawing process the electrode element 12 may be cut into shape, including for example contact elements 125 being formed at a lower edge of the electrode element 12, as visible from Fig. 6.

Because edges of the electrode element 12 are formed by curved wall sections 121, 124, a current density across the electrode element 12 - when using the electrode device 1 for example for a neuro-stimulation - may be improved in that the current density may be shaped to prevent density peaks at the edges of the electrode element 12.

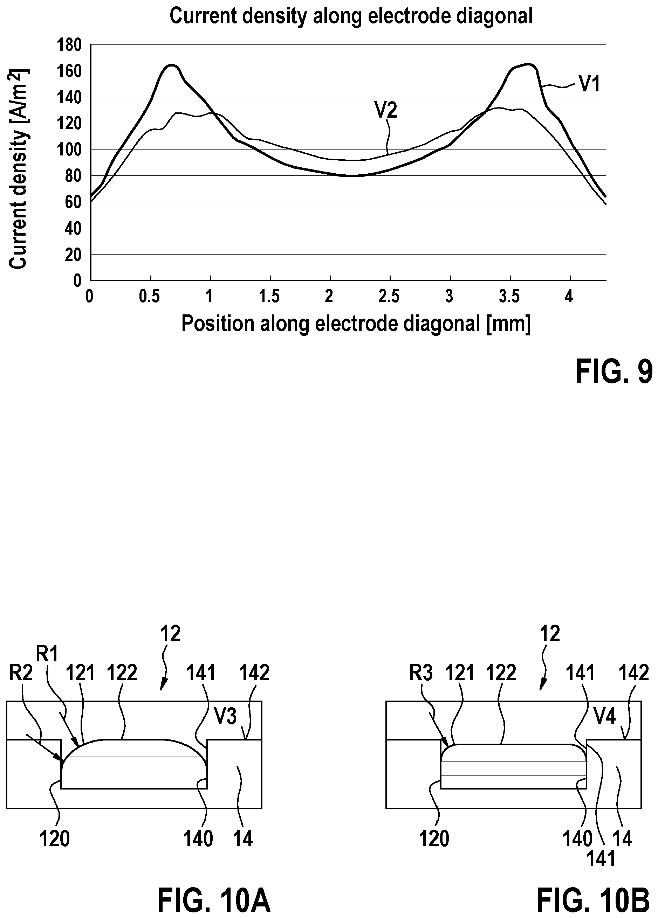

This is illustrated in Figs. 8A, 8B and Fig. 9. Herein, Fig. 9 shows current density profiles for the embodiments of the electrode element 12 as shown in Fig. 8A (version V1) and Fig. 8B (version V2).

Whereas the electrode device 12 of Fig. 8B corresponds to the embodiment of Figs. 5 to 7A to 7C, in the embodiment of Fig. 8A the electrode device 12 comprises a smaller curvature in wall sections 121, 124 defined by a radius of curvature R3, the electrode element 12 in addition protruding by a reduced height H4 beyond the surface 142 of the carrier element 14.

As visible from Fig. 9, in the electrode element 12 according to Fig. 8B (version V2) a current density profile is obtained which exhibits a reduced peak height at edges of the electrode element 12, but an increased level in a center region of the electrode element 12, in comparison to the electrode element 12 according to the embodiment of Fig. 8A (version V1).

By reducing the level of peaks in the current density profile, a local heating and damaging of tissue during a stimulation operation may be prevented, and stimulation efficiency as well as reception sensitivity may be improved.

Figs. 10A to 10F show - in cross-sectional views corresponding to the view of Fig. 5 - different embodiments of electrode elements 12 as embedded in carrier elements 14, the electrode elements 12 differing in their rounded shape and in their placement within the carrier element 14.

In the embodiment of Fig. 10A (version V3), the electrode element 12 in its shape substantially matches the embodiment according to Figs. 5 to 7A to 7C, but does not protrude beyond the surface 142 of the carrier element 14. The carrier element 14 herein forms an overlapping portion 141, which however is not arranged at an angle with respect to the perpendicular direction Z.

In the embodiment of Fig. 10B (version V4), the electrode element 12 is substantially shaped as in the embodiment of Fig. 8A, but does not protrude beyond the surface 142 of the carrier element 14. The electrode element 12 is embedded into the carrier element 14 such that the planar, horizontal face 122 of the electrode element 12 comes to lie beneath the surface 142. The carrier element 14, like in the embodiment of Fig. 10A, forms an overlapping portion 141 which is not arranged at an angle with respect to the perpendicular direction Z.

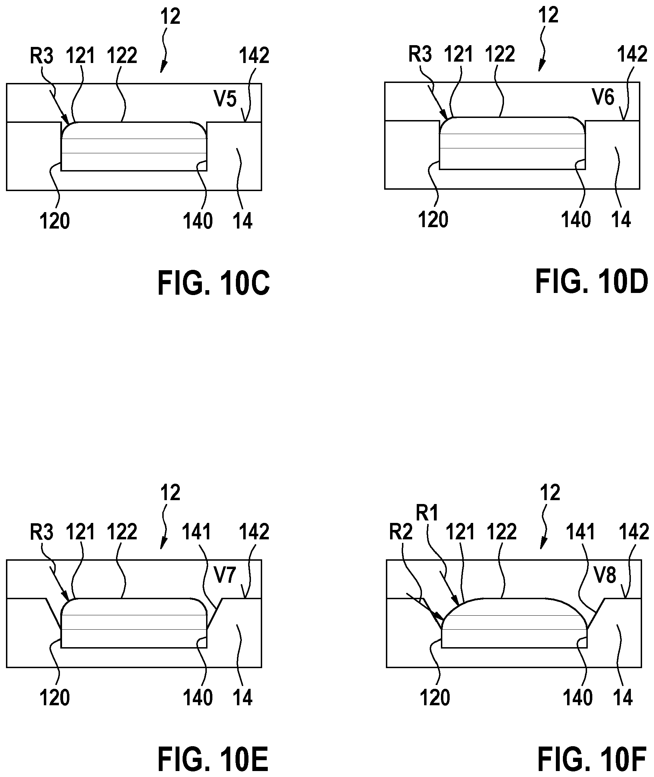

The embodiment of Fig. 10C (version V5) matches the embodiment of Fig. 10B, wherein however the horizontal wall section 122 is arranged at substantially the same height as the surface 142.

The embodiment of Fig. 10D (version V6) substantially matches the embodiment of Fig. 10C, wherein however the horizontal wall section 122 protrudes beyond the surface 142.

The embodiment of Fig. 10E (version V7) substantially matches the embodiment of Fig. 10C, wherein the carrier element 14 forms an overlapping portion 141 arranged at an angle with respect to the perpendicular direction Z.

The embodiment of Fig. 10F (version V8) substantially matches the embodiment of Fig. 10A, wherein, like in Fig. 10E, the carrier element 14 forms an overlapping portion 141 arranged at an angle with respect to the perpendicular direction Z.

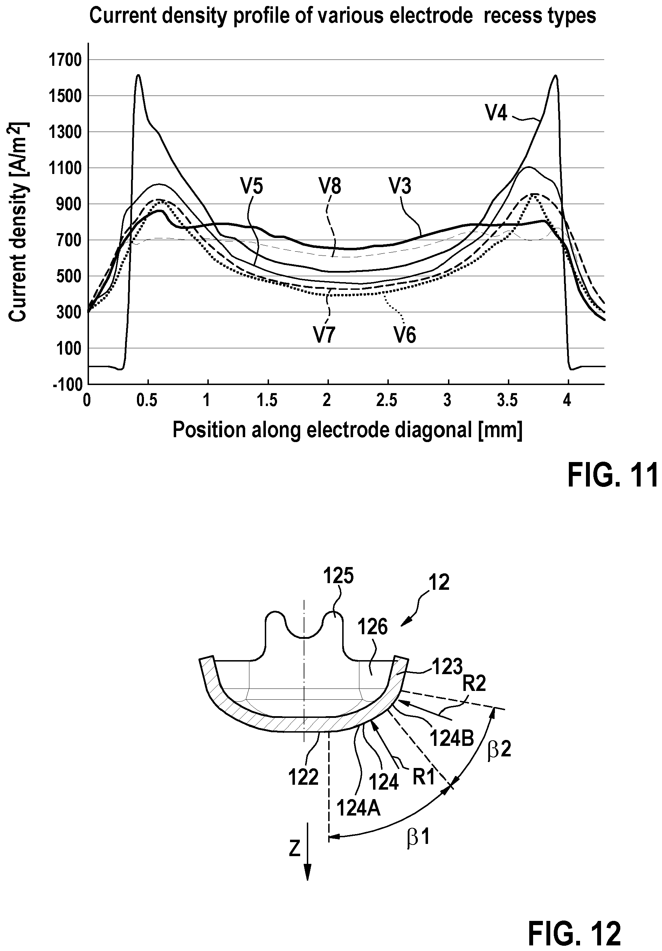

Fig. 11 shows current density profiles for the different embodiments of Figs. 10A to 10F, as measured across the electrode surface along the cross-sectional planes of Figs. 10A to 10F. For the different embodiments herein different shapes of the current density profiles are obtained, the current density profiles generally differing in peak heights obtained at the edges of the respective electrode element 12 and a level in a central region of the electrode element 12.

From Fig. 11 it generally can be concluded that a widened curvature at the edges, as in the embodiments of Figs. 10A and 10F, may yield an improved current density profile, exhibiting reduced current density peaks at the edges and a rather flattened, even density across the electrode element 12.

Fig. 12 shows another embodiment of an electrode element 12, which substantially matches the embodiment of the electrode element described above according to Figs. 6 and 7A to 7C. In comparison to the embodiment of Figs. 6 and 7A to 7C, however, at least one of the straight wall sections 120, 123 pointing inwards is arranged at an oblique angle with respect to the perpendicular direction Z, such that inner edges of the electrode element 12 are outwardly flared, as visible from Fig. 12. Hence, in this embodiment one or both of the straight wall sections 120, 123, which are in contact with the material of the carrier element 14, are not arranged along the perpendicular direction Z, but are arranged at an oblique angle, allowing for example to smooth a transition in between the sections 120, 123 and the adjoining sections 121, 124.

The idea underlying the invention is not limited to the embodiments described above, but may be implemented in a different fashion.

An electrode device as described herein may generally be used as neuro-stimulation electrode in combination with a neuro-stimulation device in the region of the spinal column or for brain stimulation. An electrode device of the type described herein however may also be used in other applications within a patient, for example for emitting stimulation signals or receiving sense signals, for example in cardiac applications.

The electrode device may comprise one or multiple electrodes elements, wherein the electrode elements may be evenly or unevenly distributed across a carrier element.

Although the carrier element has been described herein as having a generally flat shape, the carrier element may likewise have a curved shape and may extend along a curved plane of extension.

### List of Reference Numerals

- 1: Implantable electrode device
- 10: Electrode body
- 11: Electrode end
- 12: Electrode element
- 120-124: Wall section
- 121A, 121B: Curved portion
- 124A, 124B: Curved portion
- 125: Protrusion element
- 126: Recess
- 13: Supply line
- 14: Carrier element
- 140: Body portion
- 141: Overlapping portion
- 142: Surface
- 2: Stimulation device
- 20: Connector block
- α: Angle
- β1, β2: Angular width
- A: Plane of extension
- E: Epidural space
- H1-H3: Height
- L1: Length
- L2: Width
- P: Patient
- R: Spinal cord
- R1-R3: Radius
- V1-V8: Version
- W: Spinal column
- X, Y, Z: Spatial direction

## Claims

1. A medical electrode device (1) for implantation into a patient (P), comprising:
a carrier element (14) being formed from an electrically insulating material and defining a surface (142) generally extending along a plane of extension (A); and
at least one electrode element (12) arranged on the carrier element (14) for at least one of emitting an electrical signal and receiving an electrical signal;
wherein the at least one electrode element (12) comprises a first wall section (120, 123) and a second wall section (121, 124),
wherein said first wall section (120, 123), in a cross-section along a cross-sectional plane perpendicular to said plane of extension (A), extends straight along a perpendicular direction (Z) with respect to said plane of extension (A) or at an oblique angle with respect to said perpendicular direction (Z), the first wall section (120, 123) being in contact with the electrically insulating material of the carrier element (14), and
wherein said second wall section (121, 124), in said cross-section along said cross-sectional plane perpendicular to said plane of extension (A), adjoins said first wall section (120, 123) and is curved, the second wall section (121, 124) not being in contact with the electrically insulating material of the carrier element (14), and
the at least one electrode element (12) comprises a third wall section (122) adjoining the second wall section (121, 124), wherein said third wall section (122), in said cross-section along said cross-sectional plane perpendicular to said plane of extension (A), extends straight in a direction (X, Y) parallel to said plane of extension (A).

2. The medical electrode device (1) according to claim 1, wherein the at least one electrode element (12) protrudes, along said perpendicular direction (Z), from the carrier element (14) beyond said surface (142).

3. The medical electrode device (1) according to claims 1 or 2, wherein the second wall section (121, 124), in said cross-section along said cross-sectional plane perpendicular to said plane of extension (A), comprises a curved portion (121A, 121B, 124A, 124B) having an angular width (β1, β2) and a radius of curvature (R1, R2) across said angular width (β1, β2).

4. The medical electrode device (1) according to claim 3, wherein said radius of curvature (R1, R2) is equal to or larger than 0.5 mm.

5. The medical electrode device (1) according to one of the preceding claims, wherein the second wall section (121, 124), in said cross-section along said cross-sectional plane perpendicular to said plane of extension (A), comprises a first curved portion (121A, 124A) and a second curved portion (121B, 124B) having a different curvature than the first curved portion (121A, 124A), the second curved portion (121B, 124B) adjoining with said first wall section (120, 123).

6. The medical electrode device (1) according to claim 5, wherein the first curved portion (121A, 124A) comprises a first angular width (β1) and a first radius of curvature (R1) across said first angular width (β1), and the second curved portion (121B, 124B) comprises a second angular width (β2) and a second radius of curvature (R2) across said second angular width (β2).

7. The medical electrode device (1) according to claim 6, wherein the first radius of curvature (R1) is larger than the second radius of curvature (R2).

8. The medical electrode device (1) according to claim 6 or 7, wherein the first radius of curvature (R1) is equal to or larger than 0.5 mm, and the second radius of curvature (R2) is equal to or larger than 0.1 mm.

9. The medical electrode device (1) according to one of the preceding claims, wherein the carrier element (14) comprises a body portion (140) and an overlapping portion (141), wherein the body portion (141) contacts said first wall section (120, 123) of the at least one electrode element (12) and the overlapping portion (141) overlaps with said second wall section (121, 124) of the at least one electrode element (12) along said perpendicular direction (Z) without contacting the second wall section (121, 124).

10. The medical electrode device (1) according to claim 9, wherein the overlapping portion (141), in said cross-section along said cross-sectional plane perpendicular to said plane of extension (A), extends at an angle (α) with respect to the perpendicular direction (Z).

11. The medical electrode device (1) according to one of the preceding claims, wherein the at least one electrode element (12), in a cross-section parallel to said plane of extension (A), comprises a generally rectangular shape.

12. The medical electrode device (1) according to one of the preceding claims, wherein the at least one electrode element (12) is formed from a metal material in a deep-drawing process.

13. The medical electrode device (1) according to one of the preceding claims, wherein the carrier element (14) is made from a silicone material.

14. A method for fabricating a medical electrode device (1) for implantation into a patient (P), the method comprising:
forming a carrier element (14) from an electrically insulating material such that the carrier element (14) defines a surface (142) generally extending along a plane of extension (A);
forming, using a deep-drawing process, at least one electrode element (12) for at least one of emitting an electrical stimulation signal and receiving an electrical sense signal; and
arranging said at least one electrode element (12) on said carrier element (14);
wherein said forming the at least one electrode element (12) includes: forming the at least one electrode element (12) to comprise a first wall section (120., 123), a second wall section (121, 124) adjoining said first wall section, and a third wall section (122) adjoining said second wall section (121, 124);
wherein said arranging step includes: arranging said at least one electrode element (12) on said carrier element (14) such that the first wall section (120, 123) is in contact with the electrically insulating material of the carrier element (14) and the second wall section (121, 124) is not in contact with the electrically insulating material of the carrier element (14), wherein said first wall section (120, 123), in a cross-section along a cross-sectional plane perpendicular to said plane of extension (A), extends straight along a perpendicular direction (Z) with respect to said plane of extension (A) or at an oblique angle with respect to said perpendicular direction (Z), wherein said second wall section (121, 124), in said cross-section along said cross-sectional plane perpendicular to said plane of extension (A), is curved, and wherein said third wall section (122), in said cross-section along said cross-sectional plane perpendicular to said plane of extension (A), extends straight in a direction (X, Y) parallel to said plane of extension (A).

## Patentansprüche

1. Medizinische Elektrodenvorrichtung (1) zur Implantation in einen Patienten (P), umfassend:
ein Trägerelement (14), das aus einem elektrisch isolierenden Material ausgebildet ist und eine Oberfläche (142) definiert, die sich generell entlang einer Ausdehnungsebene (A) erstreckt; und
mindestens ein Elektrodenelement (12), das an dem Trägerelement (14) angeordnet ist, für mindestens eins von Emittieren eines elektrischen Signals und Empfangen eines elektrischen Signals;
wobei das mindestens eine Elektrodenelement (12) einen ersten Wandabschnitt (120, 123) und einen zweiten Wandabschnitt (121, 124) umfasst,
wobei genannter erster Wandabschnitt (120, 123), in einem Querschnitt entlang einer Schnittebene senkrecht zu genannter Ausdehnungsebene (A), gerade entlang einer senkrechten Richtung (Z) in Bezug auf genannte Ausdehnungsebene (A) oder in einem schiefen Winkel in Bezug auf genannte senkrechte Richtung verläuft, wobei der erste Wandabschnitt (120, 123) in Kontakt mit dem elektrisch isolierenden Material des Trägerelements (14) ist, und
wobei genannter zweiter Wandabschnitt (121, 124), in genanntem Querschnitt entlang genannter Schnittebene senkrecht zu genannter Ausdehnungsebene (A), an den genannten ersten Wandabschnitt (120, 123) angrenzt und gebogen ist, wobei der zweite Wandabschnitt (121, 124) nicht mit dem elektrisch isolierenden Material des Trägerelements (14) in Kontakt ist, und
wobei das mindestens eine Elektrodenelement (12) einen dritten Wandabschnitt (122) umfasst, der an den zweiten Wandabschnitt (121, 124) angrenzt, wobei genannter dritter Wandabschnitt (122), in genanntem Querschnitt entlang genannter Schnittebene senkrecht zu genannter Ausdehnungsebene (A), gerade in eine Richtung (X, Y) verläuft, die parallel zu genannter Ausdehnungsebene (A) ist.

2. Medizinische Elektrodenvorrichtung (1) nach Anspruch 1, wobei das mindestens eine Elektrodenelement (12) entlang genannter senkrechter Richtung (Z) aus dem Trägerelement (14) über die genannte Oberfläche (142) hinausragt.

3. Medizinische Elektrodenvorrichtung (1) nach Ansprüchen 1 oder 2, wobei der zweite Wandabschnitt (121, 124) in genanntem Querschnitt entlang genannter Schnittebene senkrecht zu genannter Ausdehnungsebene (A) einen gebogenen Abschnitt (121A, 121B, 124A, 124B) mit einer Winkelweite (β1, β2) und einem Kurvenradius (R1, R2) über genannte Winkelweite (β1, β2) umfasst.

4. Medizinische Elektrodenvorrichtung (1) nach Anspruch 3, wobei genannter Kurvenradius (R1, R2) gleich oder größer 0,5 mm ist.

5. Medizinische Elektrodenvorrichtung (1) nach einem der vorstehenden Ansprüche, wobei der zweite Wandabschnitt (121, 124), in genanntem Querschnitt entlang genannter Schnittebene senkrecht zu genannter Ausdehnungsebene (A), einen ersten gebogenen Abschnitt (121A, 124A) und einen zweiten gebogenen Abschnitt (121B, 124B) mit einer anderen Krümmung als der erste gebogene Abschnitt (121A, 124A) umfasst, wobei der zweite gebogene Abschnitt (121B, 124B) an genannten ersten Wandabschnitt (120, 123) angrenzt.

6. Medizinische Elektrodenvorrichtung (1) nach Anspruch 5, wobei der erste gebogene Abschnitt (121A, 124A) eine erste Winkelweite (β1) und einen ersten Kurvenradius (R1) über genannte erste Winkelweite (β1) umfasst, und der zweite gebogene Abschnitt (121B, 124B) eine zweite Winkelweite (β2) und einen zweiten Kurvenradius (R2) über die genannte zweite Winkelweite (β2) umfasst.

7. Medizinische Elektrodenvorrichtung (1) nach Anspruch 6, wobei der erste Kurvenradius (R1) größer als der zweite Kurvenradius (R2) ist.

8. Medizinische Elektrodenvorrichtung (1) nach Anspruch 6 oder 7, wobei der erste Kurvenradius (R1) gleich oder größer als 0,5 mm ist, und der zweite Kurvenradius (R2) gleich oder größer als 0,1 mm ist.

9. Medizinische Elektrodenvorrichtung (1) nach einem der vorstehenden Ansprüche, wobei das Trägerelement (14) einen Körperabschnitt (140) und einen überlappenden Abschnitt (141) umfasst, wobei der Körperabschnitt (141) genannten ersten Wandabschnitt (120, 123) des mindestens einen Elektrodenelements (12) berührt und der überlappende Abschnitt (141) mit genanntem zweitem Wandabschnitt (121, 124) des mindestens einen Elektrodenelements (12) entlang genannter senkrechter Richtung (Z) überlappt, ohne den zweiten Wandabschnitt (121, 124) zu berühren.

10. Medizinische Elektrodenvorrichtung (1) nach Anspruch 9, wobei der überlappende Abschnitt (141), in genanntem Querschnitt entlang genannter Schnittebene senkrecht zu genannter Ausdehnungsebene (A), in einem Winkel (α) in Bezug auf die senkrechte Richtung (Z) verläuft.

11. Medizinische Elektrodenvorrichtung (1) nach einem der vorstehenden Ansprüche, wobei das mindestens eine Elektrodenelement (12) in einem Querschnitt parallel zu genannter Ausdehnungsebene (A) eine generell rechteckige Form umfasst.

12. Medizinische Elektrodenvorrichtung (1) nach einem der vorstehenden Ansprüche, wobei das mindestens eine Elektrodenelement (12) aus einem Metallmaterial in einem Tiefziehverfahren ausgebildet ist.

13. Medizinische Elektrodenvorrichtung (1) nach einem der vorstehenden Ansprüche, wobei das Trägerelement (14) aus einem Silikonmaterial hergestellt ist.

14. Verfahren zur Fertigung einer medizinischen Elektrodenvorrichtung (1) zur Implantation in einen Patienten (P), das Verfahren umfassend:
Ausbilden eines Trägerelements (14) aus einem elektrisch isolierenden Material in der Art, dass sich das Trägerelement (14) eine Oberfläche (142) definiert, die sich generell entlang einer Ausdehnungsebene (A) erstreckt;
Ausbilden, mittels Tiefziehverfahren, von mindestens einem Elektrodenelement (12) für mindestens eins von einer Ausgabe eines elektrischen Stimulationssignals und einem Erhalt eines elektrischen Erfassungssignals; und
Anordnen des genannten mindestens einen Elektrodenelements (12) an genanntem Trägerelement (14), wobei genanntes Ausbilden des mindestens einen Elektrodenelements (12) umfasst:
Ausbilden des mindestens einen Elektrodenelements (12), so dass es einen ersten Wandabschnitt (120, 123), einen zweiten Wandabschnitt (121, 124), der an den genannten ersten Wandabschnitt angrenzt, und einen dritten Wandabschnitt (122), der an den genannten zweiten Wandabschnitt (121, 124) angrenzt, umfasst;
wobei genannter Anordnungsschritt umfasst: Anordnen des genannten mindestens einen Elektrodenelements (12) an genanntem Trägerelement (14) in der Art, dass der erste Wandabschnitt (120, 123) mit dem elektrisch isolierenden Material des Trägerelements (14) in Kontakt ist und der zweite Wandabschnitt (121, 124) nicht mit dem elektrisch isolierenden Material des Trägerelements (14) in Kontakt ist, wobei genannter erster Wandabschnitt (120, 123), in einem Querschnitt entlang einer Schnittebene (Z) senkrecht zu genannter Ausdehnungsebene (A), gerade entlang einer senkrechten Richtung (Z) in Bezug auf genannte Ausdehnungsebene (A) oder in einem schrägen Winkel in Bezug auf genannte senkrechte Richtung (Z) verläuft, wobei genannter zweiter Wandabschnitt (121, 124), in genanntem Querschnitt entlang genannter Schnittebene senkrecht zu genannter Ausdehnungsebene (A), gebogen ist, und wobei genannter dritter Wandabschnitt (122), in genanntem Querschnitt entlang genannter Schnittebene senkrecht zu genannter Ausdehnungsebene (A), gerade in eine Richtung (X,Y) verläuft, die parallel zu genannter Ausdehnungsebene (A) ist.

## Revendications

1. Dispositif d'électrode médicale (1) pour implantation dans un patient (P), comprenant :
un élément de support (14) façonné à partir d'un matériau électriquement isolant et définissant une surface (142) s'étendant de manière générale le long d'un plan d'extension (A) ; et
au moins un élément d'électrode (12) agencé sur l'élément de support (14) pendant au moins un parmi l'émission d'un signal électrique et la réception d'un signal électrique ;
dans lequel l'au moins un élément d'électrode (12) comprend une première section de paroi (120, 123) et une deuxième section de paroi (121, 124),
dans lequel ladite première section de paroi (120, 123), dans une section transversale le long d'un plan en section transversale perpendiculaire audit plan d'extension (A), s'étend en ligne droite le long d'une direction perpendiculaire (Z) par rapport audit plan d'extension (A) ou à un angle oblique par rapport à ladite direction perpendiculaire (Z), la première section de paroi (120, 123) étant en contact avec le matériau électriquement isolant de l'élément de support (14), et
dans lequel ladite deuxième section de paroi (121, 124), dans ladite section transversale le long dudit plan en section transversale perpendiculaire audit plan d'extension (A), rejoint ladite première section de paroi (120, 123) et est incurvée, la deuxième section de paroi (121, 124) n'étant pas en contact avec le matériau électriquement isolant de l'élément de support (14), et
l'au moins un élément d'électrode (12) comprend une troisième section de paroi (122) rejoignant la seconde section de paroi (121, 124), dans lequel ladite troisième section de paroi (122), dans ladite section transversale le long dudit plan en section transversale perpendiculaire audit plan d'extension (A), s'étend en ligne droite dans une direction (X, Y) parallèle audit plan d'extension (A).

2. Dispositif d'électrode médicale (1) selon la revendication 1, dans lequel l'au moins un élément d'électrode (12) fait saillie, le long de ladite direction perpendiculaire (Z), depuis l'élément de support (14) au-delà de ladite surface (142).

3. Dispositif d'électrode médicale (1) selon les revendications 1 ou 2, dans lequel la deuxième section de paroi (121, 124), dans ladite section transversale le long dudit plan en section transversale perpendiculaire audit plan d'extension (A), comprend une partie incurvée (121A, 121B, 124A, 124B) ayant une largeur angulaire (β1, β2) et un rayon de courbure (R1, R2) à travers ladite largeur angulaire (β1, β2).

4. Dispositif d'électrode médicale (1) selon la revendication 3, dans lequel ledit rayon de courbure (R1, R2) est supérieur ou égal à 0,5 mm.

5. Dispositif d'électrode médicale (1) selon l'une des revendications précédentes, dans lequel la deuxième section de paroi (121, 124), dans ladite section transversale le long dudit plan en section transversale perpendiculaire audit plan d'extension (A), comprend une première partie incurvée (121A, 124A) et une seconde partie incurvée (121B, 124B) ayant une courbure différente de celle de la première partie incurvée (121A, 124A), la seconde partie incurvée (121B, 124B) rejoignant ladite première section de paroi (120, 123).

6. Dispositif d'électrode médicale (1) selon la revendication 5, dans lequel la première partie incurvée (121A, 124A) comprend une première largeur angulaire (β1) et un premier rayon de courbure (R1) le long de ladite première largeur angulaire (β1), et la seconde partie incurvée(121B, 124B) comprend une seconde largeur angulaire (β2) et un second rayon de courbure (R2) à travers ladite seconde largeur angulaire (β2).

7. Dispositif d'électrode médicale (1) selon la revendication 6, dans lequel le premier rayon de courbure (R1) est plus grand que le second rayon de courbure (R2).

8. Dispositif d'électrode médicale (1) selon la revendication 6 ou 7, dans lequel le premier rayon de courbure (R1) est supérieur ou égal à 0,5 mm, et le second rayon de courbure (R2) est supérieur ou égal à 0,1 mm.

9. Dispositif d'électrode médicale (1) selon l'une des revendications précédentes, dans lequel l'élément de support (14) comprend une partie de corps (140) et une partie superposée (141), dans lequel la partie de corps (141) entre en contact avec ladite première section de paroi (120, 123) dudit au moins un élément d'électrode (12) et la partie superposée (141) se superpose à ladite seconde section de paroi (121, 124) de l'au moins un élément d'électrode (12) le long de ladite direction perpendiculaire (Z) sans entrer en contact avec la seconde section de paroi (121, 124).

10. Dispositif d'électrode médicale (1) selon la revendication 9, dans lequel la partie superposée (141), dans ladite section transversale le long dudit plan en section transversale perpendiculaire audit plan d'extension (A), s'étend à un angle (α) par rapport à la direction perpendiculaire (Z).

11. Dispositif d'électrode médicale (1) selon l'une des revendications précédentes, dans lequel l'au moins un élément d'électrode (12), dans une section transversale parallèle audit plan d'extension (A), comprend une forme généralement rectangulaire.

12. Dispositif d'électrode médicale (1) selon l'une des revendications précédentes, dans lequel l'au moins un élément d'électrode (12) est façonné à partir d'un matériau métallique dans un processus d'emboutissage profond.

13. Dispositif d'électrode médicale (1) selon l'une des revendications précédentes, dans lequel l'élément de support (14) est fabriqué en un matériau à base de silicone.

14. Procédé de fabrication d'un dispositif d'électrode médicale (1) pour implantation dans un patient (P), le procédé comprenant les étapes consistant à :
façonner un élément de support (14) à partir d'un matériau électriquement isolant de telle sorte que l'élément de support (14) définit une surface (142) s'étendant de manière générale le long d'un plan d'extension (A) ;
façonner, en utilisant un processus d'emboutissage profond, au moins un élément d'électrode (12) pour au moins une parmi l'émission d'un signal de stimulation électrique et la réception d'un signal de détection électrique ; et
agencer ledit au moins un élément d'électrode (12) sur ledit élément de support (14) ;
dans lequel ladite étape consistant à façonner l'au moins un élément d'électrode (12) inclut une étape consistant à : façonner l'au moins un élément d'électrode (12) afin qu'il comprenne une première section de paroi (120, 123),
une deuxième section de paroi (121, 124) rejoignant ladite première section de paroi, et une troisième section de paroi (122) rejoignant ladite deuxième section de paroi (121, 124) ;
dans lequel ladite étape consistant à agencer inclut une étape consistant à : agencer ledit au moins un élément d'électrode (12) sur ledit élément de support (14) de telle sorte que la première section de paroi (120, 123) est en contact avec le matériau électriquement isolant de l'élément de support (14) et la deuxième section de paroi (121, 124) n'est pas en contact avec le matériau électriquement isolant de l'élément de support (14), dans lequel ladite première section de paroi (120, 123), dans une section transversale le long d'un plan en section transversale perpendiculaire audit plan d'extension (A), s'étend en ligne droite le long d'une direction perpendiculaire (Z) par rapport audit plan d'extension (A) ou à un angle oblique par rapport à ladite direction perpendiculaire (Z), dans lequel ladite deuxième section de paroi (121, 124), dans ladite section transversale le long dudit plan en section transversale perpendiculaire audit plan d'extension (A), est incurvée, et dans lequel ladite troisième section de paroi (122), dans ladite section transversale le long dudit plan en section transversale perpendiculaire audit plan d'extension (A), s'étend en ligne droite dans une direction (X, Y) parallèle audit plan d'extension (A).
